# EUROPEAN PATENT SPECIFICATION

(11) **EP 2 122 352 B1**
(45) Date of publication and mention of the grant of the patent: **02.05.2012**
(21) Application number: 07849492.9
(22) Date of filing: 14.12.2007
(51) Int. Cl.: B82Y 5/00, B82Y 10/00, G01N 33/542, C12Q 1/68

(54) **A METHOD AND A MICRODEVICE FOR THE IDENTIFICATION AND/OR QUANTIFICATION OF AN ANALYTE IN A BIOLOGICAL SAMPLE**
VERFAHREN UND MIKROVORRICHTUNG ZUR IDENTIFIZIERUNG UND/ODER QUANTIFIZIERUNG EINES ANALYTEN IN EINER BIOLOGISCHEN PROBE
PROCÉDÉ ET MICRODISPOSITIF D'IDENTIFICATION ET/OU DE QUANTIFICATION D'UN ANALYTE DANS UN ÉCHANTILLON BIOLOGIQUE

(30) Priority: 14.12.2006 IT TO20060883
(43) Date of publication of application: 25.11.2009
(73) Proprietor: Consiglio Nazionale Delle Ricerche, Roma (IT); Fondazione Istituto Italiano Di Tecnologia, 16163 Genova (IT)
(72) Inventor: POMPA, Pier Paolo, 73100 Lecce (IT); SABELLA, Stefania, 73013 Galatina (Lecce) (IT); RINALDI, Rosaria, 73100 Lecce (IT); CINGOLANI, Roberto, 73100A Arnesano (Lecce) (IT); CALABI, Franco, 73100 Lecce (IT)
(74) Representative: Rambelli, Paolo
(86) International application number: PCT/IB2007/055112
(87) International publication number: WO 2008/072209

(56) References cited:
- WO-A-2006/066977
- WO-A-2007/078297
- WO-A2-2004/057014
- J.Y. LEE ET AL.: "Miniaturization of polymerase chain reaction" BIOTECHNOLOGY AND BIOPROCESS ENGINEERING, vol. 8, 2003, pages 213-220, XP002479528 The Korean Society for Biotechnology and Bioengineering, Seoul. cited in the application

## Description

The present invention relates to a method and a microdevice for identification and/or quantification, particularly in real time, of an analyte, particularly a biomolecule present in a biological sample, and is particularly applicable to the field of diagnostic analysis (genome and/or proteome analysis) and to the production ofbiochips.

Over the last ten years, there has been a considerable proliferation of biochips, and numerous terms have been used to denote them (for example "gene-chip", "gene-array", "DNA micro-array", "protein chip", "lab-on-a-chip"). Essentially, these chips, whether developed in simple formats or integrated into more complex devices or architectures, are planar structures of various materials (commonly glass or plastics), on which (bio)molecules such as DNA, protein and cells capable of selectively recognizing target molecules are immobilized (by chemical modification of the surface or by in situ synthesis) [Fan, 2006].

Biochip technology has revolutionized molecular biology, and is widely used for studying gene and protein expression (genomic and/or proteomic expression) in various fields such as experimental diagnosis and clinical medicine, the discovery of new medicines (biomarker discovery) and pharmacogenomics.

Different types of chip have been developed for a very wide range of applications such as enzyme assays [Hadd, 1997], immunochemical assays [Wang, 2001], analysis for recognition of polymorphism in gene variants [Dunn, 2000], and nucleic acid sequencing [Scherer, 1999], together with chips for carrying out DNA ligation and amplification reactions on microvolumetric scales (the ligase chain reaction [Cheng, 1996] and the polymerase chain reaction (PCR) [Kopp, 1998; Daniel, 1998]).

In particular, because of the high specificity of the hybridization reaction between oligonucleotide sequences (selective pairing of A-T, G-C), DNA chips have been developed more rapidly than protein chips. Although the market for the latter is enormously valuable to the scientific world, its development has been slower than that predicted initially for this technology, because of the complexity of the interactions underlying the biorecognition mechanism between protein molecular species [Bodovitz, 2005].

In general, in a chip the event of recognition between the (bio)molecule and the target species must be translated into a detectable and measurable signal. The physical transducers for converting the signal can be of different types, based on different operating mechanisms, including optical, electrical and electrochemical transduction, or devices sensitive to variations of mass, current or frequency [Wang, 2002; Murphy, 2006].

Optical sensors therefore play a central role in the large field of biosensors. Optical detection is essentially based on the measurement of the fluorescence which develops at the biorecognition site following the event of binding between the interacting species (between complementary oligonucleotides, for example). Typically, in the most common biochip formats, one of the two biomolecular species is conjugated with a fluorescent marker.

The first optical transducers used fairly simple optical detection systems: typically, the fluorescence signal was derived from a fluorophore conjugated with the target biomolecule, and the signal was detected at the end of the hybridization (biorecognition) reaction. These approaches can be used for the purpose of purely qualitative detection, concerning the presence or absence of the target biomolecule in the solution being examined (the "on/off" approach), but cannot be used to obtain real-time (quantitative) data on the analyte interaction events.

A number of innovative optical detection systems were subsequently developed to provide greater sensitivity and flexibility in the detection system. For example, the use of optical fibres for signal transduction has become widespread, new detection strategies (such as FRET, molecular beacons, TIRF, SPR, phosphorescent probes and beads) have been developed, and new fluorescent markers (for example, PicoGreen) have been designed. These strategies have yielded considerable improvements in respect of the signal detection limits [Piunno, 1995; Ferguson, 1998], but they do not provide a means of quantitative determination of the biomolecules concerned, especially in planar formats, such as microarrays, in which specific probes for biorecognition have to be immobilized on a solid substrate.

CN 1 358 867 describes a "gene chip" device which uses the FRET (Fluorescence Resonance Energy Transfer) phenomenon for the detection of nucleic acids, where the FRET phenomenon is caused by the resonant energy exchange between a donor fluorophore and an acceptor fluorophore, one of which is bound to the probe species while the other is bound to the target species.

WO2006/066977 describes the use of FRET resonance for protein detection, in which a marked protein, comprising an energy donor marker and at least one acceptor group which can accept energy from the donor marker by Förster energy transfer, is exposed to incident electromagnetic energy in order to excite the donor group and measure the fluorescence emission of the donor.

WO2007/078297 A discloses a method to identify a target analyte using a binding partner attached to a substrate coated with luminescent metal oxide particles, capable of FRET.

One object of the present invention is to provide a method and a new optical transduction device which use the FRET phenomenon, and which enable the target molecular species to be measured quantitatively in real time.

A specific object of the invention relates to the possibility of quantitatively analysing the DNA amplification process (Polymerase Chain Reaction, PCR), since this technology, in its different versions (reverse transcriptional, RT-PCR; multiplex PCR; real-time PCR [Speers, 2006; Wrong, 2005]), combined with new methods of automated synthesis of nucleic acids, provides an important research tool for diagnosis and has been widely used in genotyping and the phenotype expression of pathogenic or viral antigens [Cockerill, 2003; Domiati-Saad, 2006].

The operating principle of real-time PCR is typically based on the measurement of a fluorescent marker, whose signal increases proportionally to the quantity of DNA amplified in each reaction cycle. As a general rule, use is made of probes suitably conjugated with fluorescent molecules having different operating mechanisms (SYBR Green, TaqMan, Molecular Beacons, Scorpions, Sunrise) [Wrong, 2005].

Various examples of real-time PCR on chips are currently available. The miniaturization of this technology yields considerable advantages such as the higher speed of the thermal cycles, a significant reduction in the consumption of reagents and biological samples, the portability of the microdevice and the intrinsic possibility of use for diagnostic applications at the place of treatment [Lee, 2003].

In view of the aforesaid objects, the invention proposes a method, a device and equipment as defined in the following claims.

In one embodiment of the invention, the probe molecules are fixed to a support coated with a film comprising a polymer matrix, in which the fluorescent nanocrystals are dispersed, or a polymer matrix comprising or formed from one or more photoluminescent polymers, in which the said nanocrystals or the said photoluminescent polymer can induce a FRET phenomenon with the fluorophore; the coating film is then irradiated with a radiation having a wavelength such that it selectively excites the nanocrystals or the photoluminescent polymer, but not the fluorophore bound to the target analyte.

Alternatively, the probe molecule can be fixed to a polymer coating film comprising an electroluminescent polymer; in this case, the electroluminescent polymer is excited electrically by applying a potential difference to the polymer film.

The fluorescence signal induced by FRET is then detected in the spectral emission region of only the fluorophore which is bound to the target analyte.

Other advantages and features of the method and device according to the invention will become clear from the following description which is given with reference to the appended drawings, in which:
- Figure 1 is a general diagram of the optical transduction device according to the invention for the real-time quantitative analysis of biomolecules for genomics or proteomics;
- Figures 2A and 2B show a diagram of the device and of the corresponding optical detection system for the real-time monitoring of biomolecular interactions for genomic and/or proteomic analysis, with particular reference to the case in which the biomolecule to be analysed is DNA (real-time PCR); in particular, Figure 2B shows the absorption spectrum of the nanocrystals (NCs) and of the organic fluorophore (Cy3) used in the following example of embodiment, with an indication of the wavelength used for the exciting radiation and of the wavelength used for detection;
- Figure 3 is a general diagram of the microdevice according to the invention and of the corresponding optical detection system for the real-time monitoring of biomolecular interactions for genomic and/or proteomic analysis;
- Figure 4 is an image of a prototype microreactor according to the invention;
- Figure 5 shows an agarose gel electrophoresis of the plasmid pMPSV-RM1, amplified in 5 µl of PCR reaction mixture in a microreactor according to the invention; and
- Figure 6 is a diagram showing the typical variation of the fluorescence signal in the microdevice according to the invention (using to the optical system of Figure 2) for the real-time monitoring of the biomolecular interactions for genomic and/or proteomic analysis, where the detected signal is that of the fluorophore conjugated with the biomolecule in question following the interaction with the corresponding recognition site (excitation by FRET).

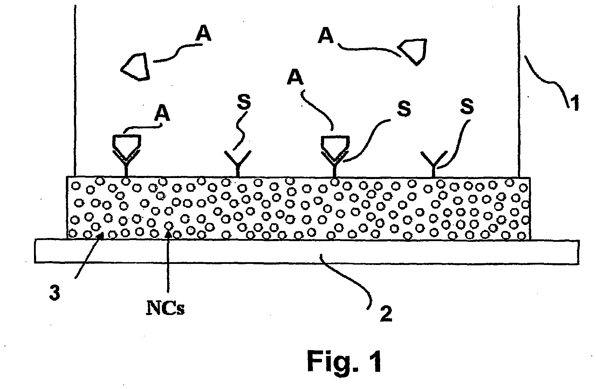

The optical transduction device proposed by the invention for the real-time quantitative determination of biomolecules, adaptable to genomic and proteomic analysis, comprises a reaction microchamber 1, intended to receive a solution containing the biological sample including the analyte or analytes A to be detected; the reaction microchamber comprises a wall 2, formed by a solid substrate coated, or at least partially coated, with a thin film 3 of polymer material (Figure 1).

The fluorescent nanocrystals NCs are uniformly dispersed in this film, and specific probes S for the analytes to be determined are immobilized on its surface.

The analytes A present in the biological sample to be detected and quantified are marked directly (by synthesis in the microchamber for example), or indirectly, with one or more suitably selected fluorophores. The specific interaction between an analyte biomolecule present in the sample and the probes immobilized on the optically active wall of the microchamber is detected by a FRET process between the NCs (which act as donors) and the fluorophores bound to the biomolecule (which act as acceptors).

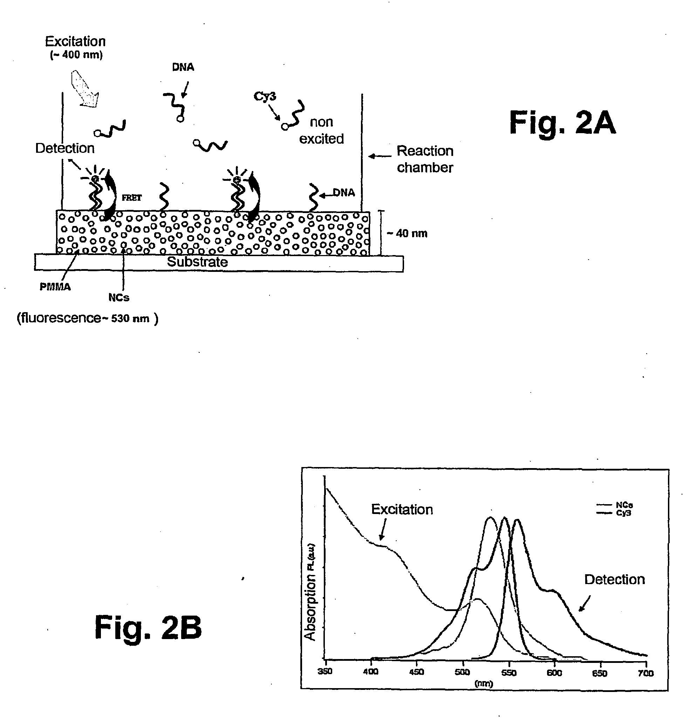

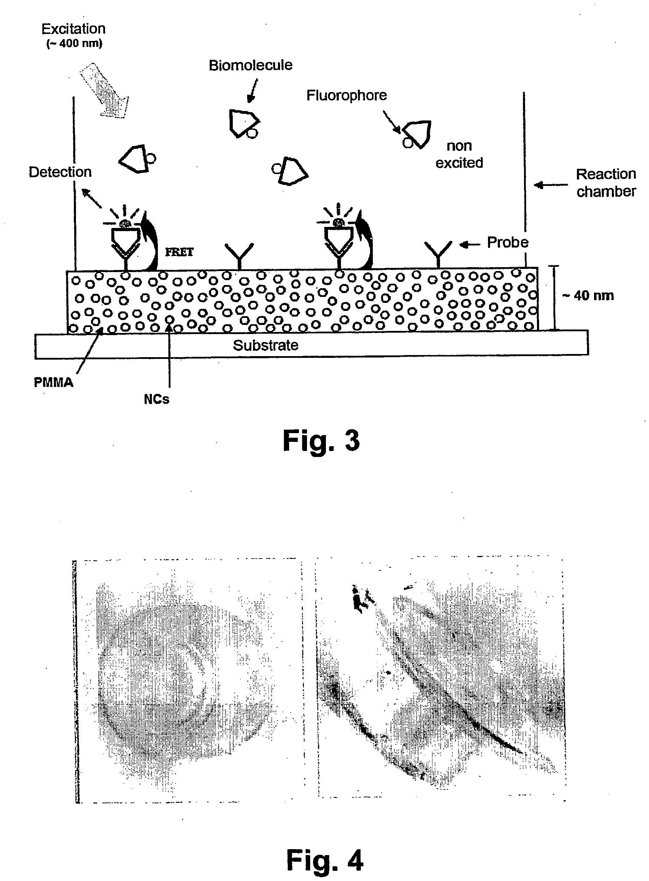

The optical system is shown in Figures 2A, 2B and 3, which represent, respectively, the case in which the device proposed by the invention is used for analysing specific nucleic acid sequences (real-time PCR) and the general case in which the quantitative determination of a biomolecule (proteins, ligands, etc.) is to be conducted.

In the first case, the specific optical parameters used in the microdevice are shown in the drawing, as a non-limiting example.

The system exploits the specific photophysical characteristics of FRET processes: the efficiency of the donor-acceptor interaction is strongly dependent on the relative distance (d) between the two species (being typically a function of d⁻⁶), and consequently there can be resonant transfers of excitation energy from the NCs to the target fluorophores only for distances which are typically less than 10 nanometres.

In the optical detection system integrated into the microdevice, these photophysical characteristics are such that an efficient process of energy transfer from the NCs to the fluorophores bound to the target molecules can be established only at the point where these biomolecules have interacted specifically with the corresponding probe (except for a negligible background which is constant in time and which is due to the biomolecules which are statistically in solution in the region within the first 10 nm from the surface of the polymer film.

By exploiting the specific spectral properties of the nanocrystals (essentially the very broad absorption spectrum; see for example the spectrum of Figure 2B), it is possible to monitor the biomolecular interactions in question by using a low-wavelength exciting radiation (typically UV/blue; for example, λ = 400 nm has been chosen in Figure 2B), which efficiently excites NCs dispersed in the polymer film, but not the target fluorophores, and by acquiring the fluorescence signal in the spectral emission region of only the fluorophores bound to the analytes to be determined (for example, at about 630-650 nm as shown in Figure 2).

The essence of this approach is that none of the free biomolecules in solution are excited, since they cannot directly absorb the exciting radiation, and therefore they make no contribution to the fluorescence signal which is to be detected. Thus this feature enables the biomolecular interactions with the specific probes to be monitored selectively in real time and in a quantitative way, and also makes it possible to analyse and quantify the biomolecules which are of interest for genomics and/or proteomics.

The nanocrystals used in the field of the invention are preferably colloidal semiconductor nanocrystals, preferably of the core-shell type; for example, CdSe/ZnS core-shell nanocrystals can be used. Examples of other materials which are frequently used to form the core are ZnSe, CdS, and CdTe. Clearly, a wide range of other materials can also be used.

However, the invention is not to be interpreted as being limited to a specific choice of fluorescent nanocrystals.

In one embodiment of the invention, the thin film of polymer material can comprise two or more types of fluorescent nanocrystals, having different spectral absorption characteristics.

The fluorophores bound to the biomolecules can be common commercial organic fluorophores or any other type of novel fluorophore, including NCs, oligothiophenes, GFPs (Green Fluorescent Proteins), beads, and hybrid systems), provided that they have suitable characteristics to act as efficient acceptor species in FRET processes using NCs dispersed in the polymer matrix (essentially, they must have a good spectral overlap with the NCs used).

The emission of the fluorophores bound to the target molecules can range over a wide spectral interval (from blue to infrared), according to the specific biomolecular application.

The analytes included in the biological sample to be analysed can be marked with different fluorophores which emit in distinct spectral regions; thus the possibility of using different NC-fluorophore pairs as donor-acceptor pairs enables analyses to be conducted in parallel on different analytes (multiplexing).

For this purpose, it is simply necessary to monitor the emission of the different fluorophores simultaneously, acquiring the different signals in different spectral windows.

Clearly, the method according to the invention is not limited to the detection and quantification of specific analytes; the analytes to be determined can, for example, comprise molecules of DNA, proteins and ligands which can be marked with one or more fluorophores.

These analytes (target compounds) can be present in biological or non-biological samples, such as clinical samples extracted from blood, urine, faeces, saliva, pus, serum, tissue, fermentation solutions or culture media. The analytes (target compounds) can preferably be isolated, purified, split, copied and/or amplified, if necessary by using methods known in the prior art.

Similarly, the specific probes immobilized on the surface of the thin polymer film can comprise specific sequences of ssDNA, antibodies, receptors, aptamers, etc.

The optically active film integrated in the microdevice according to the invention is preferably made from a solution of NCs and a polymer, preferably an elastomer, which is deposited on the solid substrate, preferably by spin-coating or by other deposition techniques which can ensure a uniform distribution of NCs in the polymer matrix; typically, the thickness of the polymer film varies from a few nanometres to several tens of microns, for example from 1 nm to 50 µm; preferred thicknesses are of the order of 10 to 100 nm.

The polymer material used must not be optically active (in the absence of NCs, it must have zero or very low intrinsic fluorescence), must be transparent in the spectral range from the near UV to the visible and to the infrared, and must have chemical and physical characteristics enabling NCs to be dispersed uniformly without significantly perturbing the specific optical properties of the film, and chemical characteristics of the surface which allow chemical functionalization with molecules capable of biorecognition.

In one embodiment of the invention, it is possible to use a polymer material with electronic resist properties (such as PMMA) which enables the optically active surface of the device to be patterned by lithographic methods.

A possible alternative to the use of an optical film formed by an inert polymer in which colloidal nanocrystals are dispersed is the use of an optical film composed of, or comprising, other active materials, such as photoluminescent or electroluminescent polymers or phosphorescent compounds, which may optionally be incorporated in polymer matrices, on which the sequences of probe DNA, or more generally (bio)molecules with the function of specific recognition of target analytes, are fixed in a similar way to that described above.

The operating mechanism (for molecular recognition of analytes by solid state FRET processes) is entirely identical to what has been described for the case in which colloidal nanocrystals are used. In particular, in the case of photoluminescent polymers, the device operates in the same way as that specified for the hybrid PMMA-NCs system, used in the following example of embodiment: the exciting radiation is selected so as to excite only the photoluminescent polymer and not the fluorophores conjugated with the target biomolecules; the latter are excited by the polymer by means of FRET processes only as a result of the biorecognition event. In the case of electroluminescent polymers, however, there is no need for an external excitation source; the polymer is excited (causing the excitation of the fluorophores conjugated with the target biomolecules via FRET, as a result of biorecognition) by an electrical method (electroluminescence), by applying a suitable potential difference to the polymer film (according to the conventional geometries and configurations of the LED devices). In the latter case, evidently, the device has the further advantage of not requiring an external excitation source, but in all other respects it operates in an identical way to the system described in relation to the use of nanocrystals.

The following examples of luminescent substances can be cited:
- photo/electroluminescent polymers:
- conjugated polymers, for example: cis- and trans-polyacetylenes, polydiacetylenes, polyparaphenylenes, polypyrroles, polythiophenes, polybithiophenes, polyisothianaphthalenes, polyphenylenevinylenes, polythienylvinylenes, polyphenylene sulphides, polyanilines e polyfluorenes;
- phosphorescent compounds, for example: iridium complexes such as FIrpic (btpy)-2-IR(acac), typically incorporated in polymer matrices, for example poly(9-vinylcarbazole) (PVK);
- short molecules, alone or incorporated in suitable matrices;
- dendrimers.

The substrate which acts as the support for the polymer matrix does not have to meet specific requirements, except that it must be physically and chemically compatible with the polymer material used; for example, silicon, silica, glass, quartz or plastics materials can be used.

The reaction chamber can be made from various materials, such as polymer or glass materials, which may or may not be suitably functionalized and passivated, provided that they are compatible with the integrated polymer matrix and with the solution containing the sample and the reagents, which is usually an aqueous solution.

The chamber can be produced in various ways, for example by etching, embossing, moulding or any other suitable manufacturing method. A suitable system of heaters and/or coolers can be integrated into the reaction chamber, to ensure that a controlled temperature of the solution in the chamber can be maintained, or that specific thermal cycles can be executed according to the application concerned.

A preferred material for the microchamber at the present time is polydimethylsiloxane (PDMS), which is currently one of the most widely used plastics materials for the production of chips for stand-alone or integrated systems. PDMS has a number of advantages: it is biologically inert, non-toxic, gas-permeable, and inexpensive, and adheres easily to other materials such as glass, polystyrene and PMMA, thus making it possible to construct hybrid systems composed of different materials.

However, the high hydrophobicity and low chemical reactivity of the groups exposed on the surface considerably limit its use in many applications [Xiang, 2005]. To improve the hydrophily of the surface, PDMS can be subjected to surface modification treatments using physical and chemical methods.

For this purpose, PDMS can be treated with oxygen plasma in experimental conditions in order to produce a minor modification of the surface (hydrolysis of the exposed methoxyl groups) rather than true etching. The action of the oxygen plasma is effective, resulting in very low typical contact angles of about 12.5 ° ± 6.25 ° measured after treatment.

In order to improve the stability of this treatment over time and prevent the treated PDMS surfaces from returning in a short time to a hydrophobic state (for example, for periods in excess of 24 hours), surface passivation procedures can be carried out. A preferred passivation procedure is a treatment with an acid solution of diamine-PEG which enables good wettability characteristics to be obtained for periods of more than two weeks.

The surface of the optically active thin film can be functionalized by known conventional methods for biochip preparation. In general, it is possible to use reactions of hydrolysis or aminolysis of the exposed free methoxyl groups and activation of the resulting groups with reactive chemical groups (mono-linkers and/or bi-linkers) for the (bio)molecules, such as oligonucleotides, proteins and peptides, with which the surface is to be functionalized.

One preferred method is to fix biological molecules such as proteins, peptides or nucleic acid sequences by a chemical bond with amine groups present on the optically active surface, bound to activating mono-linkers such as glutaraldehyde.

The insertion of amine groups (which in turn bind the activated groups on the surface) at the terminal ends of the nucleic acid chain is easily carried out by synthesis using aminated nucleotides.

It is to be understood that the probe molecules can be bound to the optically active surface in a matrix arrangement, according to known methods of biochip production.

### Working example

A prototype microreactor for real-time optical detection for genome and/or proteome analysis was constructed, using PDMS as the material for the microchamber. In order to improve the hydrophily characteristics of the surface, the PDMS was initially treated with oxygen plasma, as mentioned above, to cause a minor modification of the surface with hydrolysis of the exposed methoxyl groups.

Surface passivation procedures were also carried out, using acid solutions of diamine PEG, to increase the stability of the treatment time. This procedure was successful, and the device showed good wettability characteristics for more than two weeks.

The PDMS microreactor was constructed by pouring a solution of PDMS and polymerizing agent, in suitable concentration ratios, in circular metal moulds whose dimensions impart a constant shape and thickness to the microreactor. Following the polymerization of the solution, carried out in a furnace at 140°C for 30 minutes, the PDMS microchamber was removed from the metallic mould and cooled.

The resulting microchambers had dimensions suitable for volumes from 50 µL to 5 µL; a typical prototype microchamber is shown in Figure 4.

The possibility of producing hybrid systems, composed of different polymer materials, was also investigated, and a PDMS microreactor was constructed with a base composed of a layer of PMMA functionalized according to the procedures described below. Various PDMS sheets were also produced for the purpose of sealing the whole system and thus preventing the evaporation of solvents, since it is very important to control this phenomenon in PCR processes.

For the construction of the prototype, PMMA was selected as the polymer matrix for dispersing the nanocrystals, since it conforms to the chemical and physical characteristics described above. Various substrates (glass, silica and PDMS) were initially used as the support for the polymer matrix, and the characteristics of chemical and physical compatibility with a PMMA solution were investigated for each of these, following deposition by spin-coating. Samples of PMMA with and without colloidal semiconductor nanocrystals (standard samples and optically active samples) were prepared, and the resulting film was characterized using a profile gauge, atomic force microscopy (AFM) and confocal microscopy.

The characterizations which were carried out showed that the resulting polymer film had excellent uniformity. Additionally, the analysis of the NC emission spectra showed that the nanocrystals were dispersed uniformly in the film and that they had spectral characteristics identical to those measured in newly synthesized standard NC solutions.

In particular, in this specific working example, the thickness of the PMMA layer in which the NCs were dispersed was optimized to about 40-50 mm, since the fluorescence signals measured in these conditions were sufficiently intense for optimal detection.

As mentioned above, it is to be understood that films with different thicknesses can be used, and therefore the choice of the thickness of the optically active film is not particularly critical. In the case under consideration, CdSe/ZnS core-shell NCs were used, emitting at 530 nm; polymer films with the same optical and uniformity characteristics were also produced with NCs of different dimensions which emitted in a range from the blue to the near infrared.

NCs were dispersed in a solution of PMMA-950K (typically with the following final chlorobenzene concentrations: C_{NCs} = 7 x 10⁻⁶ M and C_{PMMA} = 1.9 x 10⁵ M). The resulting dispersed solution was deposited on the substrate by spin-coating (typical parameters: 3000 revolutions per 40 seconds) and the polymerization of the PMMA was then induced by heating on a plate at 180°C for two minutes.

In the example described here of a microdevice for quantitative determination of oligonucleotide sequences (real-time PCR), the PMMA film, made optically active by NCs dispersed in them, was functionalized by aminolysis with a single layer of primary amine groups. These groups were conjugated with oligonucleotides with terminal amine functionality by means of a linker such as glutaraldehyde.

ssDNA molecules immobilized on the PMMA form the complementary target DNA biorecognition elements which represent the product of the amplification chain reaction (PRC) conducted in the reaction chamber located above the PMMA. All the experimental conditions, for both the aminolysis reaction and the reactions of activation with glutaraldehyde and hybridization with ssDNA, were suitably optimized.

The density of the resulting primary amine groups was evaluated by reaction with the reagent fluorescein-5-isothiocyanate (FITC). The concentration of exposed amine groups was estimated to be 2-3 picomoles/cm² (in accordance with the published data [Patel, 2006]).

After the activation of the PMMA layer, which was aminated with glutaraldehyde, the density of bound molecules per cm² of surface was also quantified with ssDNA, using ssDNA marked with Cy3 and measuring the fluorescence: a concentration of 0.3-0.5 picomole/cm² was found for the probes.

A similar reaction can be used not only for DNA but also for the functionalization of the surface of PMMA or other polymer materials with molecules of different kinds and for different applications.

In order to evaluate the operation of the resulting microreactor for real-time PCR applications, a plasmid of interest (pMPSV-RM1) was initially amplified with PCR at different reaction volumes (25, 12, 6 e 3 µL). In this case, the microreactor was treated in advance by various passivation procedures (using BSA solutions, for example) and the detection was carried out at the end point of the reaction with agarose gel electrophoresis.

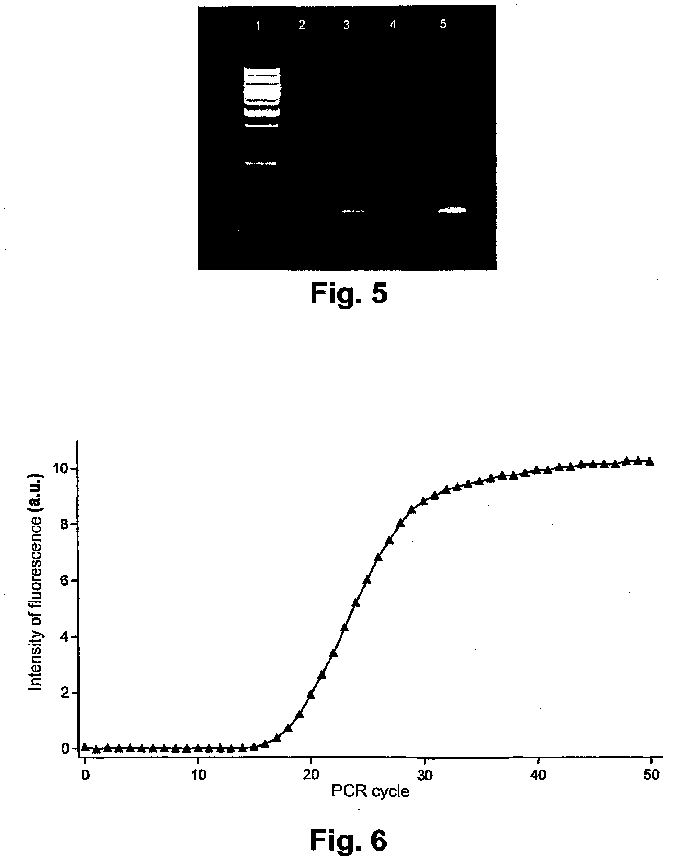

The results show that PCR takes place in the microreactor even at minimum reaction volumes (Figure 5).

In Figure 5, the light bands in lanes 3 and 5 correspond to the amplified DNA whose dimensions are comparable to those of the reference marker at 250 bp:
- lane 1: markers;
- lane 2: standard sample in microreactor without DNA template;
- lane 3: sample in microreactor with DNA template;
- lane 4: standard sample in conventional test tube without DNA template;
- lane 5: sample in conventional test tube with DNA template.

The real-time detection of the hybridization event between the two species (immobilized ssDNA and target DNA) was then conducted.

The target DNA was marked with commercial organic fluorophore (Cy3) which has a large spectral overlap with the NCs used and enables the optical detection of the interaction to be conducted beyond 600 nm (the region in which selected NCs do not emit).

The optical detection was then carried out by measuring the fluorescence signal of the Cy3 marker present in the amplified target DNA (as shown in the general diagram in Figure 2A) after the interaction with ssDNA (the complementary probes immobilized, as described above, on the optically active PMMA film).

The Cy3 fluorescence signal is produced by exciting the microreactor at a low wavelength, specifically λ ≈ 400 nm, which selectively and efficiently excites the NCs but not the fluorophores conjugated with the free biomolecules in solution, and then transferring energy (FRET) from the NCs present in the PMMA to the fluorophore conjugated with the target DNA (which has interacted with the probe DNA).

In this case, the detected fluorescence signal increases as a function of the increase in the number of DNA amplification cycles (PCR cycles), as can be observed in the signal measured in real time for the amplification of the plasmid pMPSV-RM1 (Figure 6). The resulting curve can be used to obtain quantitative information on the DNA sequences concerned.

Briefly, the method and device according to the invention have the following innovative and advantageous features:
- according to the innovative general optical system, the optically active element (the polymer film) is an integral part of the device and is not a fluorophore which is conjugated with the biomolecules or with the biorecognition element;
- the possible applications of the system are very wide-ranging: they vary from real-time PCR (without depending on the design of specific oligonucleotide sequences such as molecular beacons; in principle, the system can be used to detect any DNA sequence) to the detection of a very broad class of biomolecular interactions in genomics and/or proteomics, including the quantitative determination of proteins, ligands, etc.;
- the device and the method according to the invention have excellent characteristics of efficiency and sensitivity:
   i) the efficiency of the optical transduction signal is due to the fact that the excitation of the fluorophore by FRET processes takes place not by a single donor-acceptor interaction, but by multiple interactions between the various NCs dispersed in the polymer (near the biorecognition site) and the acceptor fluorophore conjugated with the target biomolecule; furthermore, it has been amply demonstrated in the literature that colloidal NCs are excellent donor species (in terms of efficiency) in FRET processes;
   ii) the high sensitivity of the method and of the device is due to the fact that, in addition to the acquisition of fairly strong fluorescence signals from the target molecules, the background signal (noise) is practically zero, since the exciting radiation cannot excite any species in the reaction chamber (with emission in the spectral window used for detection) except the target biomolecules which have interacted specifically with the biorecognition site;
- the method and the device can be used not only to analyse individual biomolecular species, but also to carry out quantitative determinations in parallel, using multiplexing procedures (by selecting n NC-fluorophore pairs as donor-acceptor species in FRET processes, it is possible to monitor n biomolecular species of interest, using a single excitation wavelength and acquiring the emission in different spectral regions).

Furthermore, since a material with electronic resist properties (such as PMMA) is used as the polymer material for making the optically active film of the device, the film can be patterned by lithographic methods (such as e-beam lithography) with resolutions down to a few nanometres.

Thus this approach makes it possible to obtain, with high precision and very high resolution, matrices of pixels (each pixel being a different optical transduction element) for quantitative analyses ofbiomolecules in parallel.

### REFERENCES

1) Bodovitz S., Joos T., Bachmann J., DDT, 2005, 10 (4), 283-287
2) Cheng J, Shoffner MA, Mitchelson KR, et al. J. Chrom. A 1996, 732 (1): 151-158
3) Cockerill F. R. Arch. Pathos. Lab. Med. 2003, 127, 1112-1120
4) Daniel JH, Iqbal S, Millington RB, et al. Sensors and Actuators a-Physical, 1998, 71 (1-2): 81-88
5) Domiati-Saad R., Scheuermann R..H. Clinica Chimica Acta, 2006, 363, 197-205
6) Dunn WC, Jacobson SC, Waters LC, et al. Anal. Biochem. 2000, 277 (1): 157-160
7) Fan J-B., Chee M. S., Gunderson K.L. Nature Review Genetics, 2006, 7, 632-644
8) Ferguson, J. A., Boles, T. C., Adams, C. P., Walt, D. R. Nature Biotech., 1996, 14 (13): 1681-1684
9) Hadd AG, Raymond DE, Halliwell JW, et al. Anal. Chem. 1997, 69 (17): 3407-3412
10) Kopp MU, de Mello AJ, Manz A. Science 1998, 280 (5366): 1046-1048
11) Lee J.Y., Kim, J.J., Park, T.H. Biotechnology and Bioprocess Engineering 2003, 8, 213-220
12) Murphy L. Current Opinion in Chemical Biology, 2006, 10, 177-184
13) Patel S. et al, 2006, Biomaterials, 27, 2890-2897
14) Piunno Pae, Krull Uj, Hudson Rhe, et al. Analytical Chemistry 1995 67 (15): 2635-2643
15) Scherer JR, Kheterpal I, Radhakrishnan A, et al. 1999, Electrophoresis 20 (7): 1508-1517
16) Speers D. J. Clin Biochem., 2006, 27, 39-51
17) Thiel, A., Frutos, A., Jordan, C., Corn, R., Smith, L. Anal. Chem. 1997, 69, 4984-4956
18) Wang J. Nucleic Acid Research, 2002, 28, 16, 3011-3016
19) Wang J., Ibanez A, Chatrathi M.P., Escarpa A., Anal. Chem. 2001, 73,5323-5327
20) Watts, H., Yeung, D., Parkers, H. 1994, Anal. Chem. 67, 4283-4289
21) Wrong M., L., Mediano J. F. BioTechniques, 2005, 39, 75-85
22) Xiang Q., Xu, B., Fu, R., Li, D., Biomedical Microdevices, 2005, 7:4, 273-279

## Claims

1. A method for the identification and/or quantification of a target analyte present in a sample, particularly a biological sample, comprising the operation of bringing said target analyte, bound to a fluorophore, into contact with a probe molecule immobilized on a support, to enable a specific bond to be formed between said target analyte and said probe molecule, **characterized in that** said probe molecule is fixed to the surface of a support coated with a film comprising a luminescent substance capable of inducing a phenomenon of resonant energy transfer (FRET) with said fluorophore, wherein said film comprising a luminescent substance is selected from a polymer matrix in which fluorescent nanocrystals are dispersed, a polymer matrix in which fluorescent compounds are incorporated, a film comprising a photoluminescent polymer and a film comprising an electroluminescent polymer and additionally comprising the operations of selectively exciting said substance of the support coating film, but not the fluorophore bound to said target analyte, thereby to cause the luminescence of said substance and to induce said phenomenon of resonant energy transfer with said fluorophore, and detecting the fluorescence signal induced in the spectral emission region of only the fluorophore bound to the target analyte.

2. A method according to Claim 1, wherein said luminescent substance comprises fluorescent nanocrystals dispersed in a polymer matrix, **characterized in that** the said polymer matrix is formed by a polymer material which is not optically active and is transparent in the spectral field from the near UV to the infrared.

3. A method according to Claim 2, **characterized in that** the said polymer matrix including fluorescent nanocrystals is formed by a polymer material with electronic resist properties.

4. A method according to any one of Claims 2 or 3, **characterized in that** the said polymer matrix comprises polymethyl methacrylate (PMMA).

5. A method according to any one of Claims 2 to 4, **characterized in that** the said fluorescent nanocrystals comprise colloidal semiconductor nanocrystals, preferably of the core-shell type.

6. A method according to any one of Claims 2 to 5, **characterized in that** the said fluorophore has a spectral overlap with the said nanocrystals.

7. A method according to any one of Claims 2 to 6, **characterized in that** the said polymer film comprises two or more types of nanocrystals having distinct spectral characteristics of fluorescent emission.

8. A method according to Claim 7, **characterized in that** the sample subjected to analysis comprises target analytes marked with a plurality of fluorophores which emit in distinct spectral regions, and in which the said coating film comprises a corresponding plurality of types of fluorescent nanocrystals, selected in such a way that each type of nanocrystal is capable of inducing a phenomenon of resonant energy transfer (FRET) with a corresponding fluorophore.

9. A method according to Claim 1, **characterized in that** the said electroluminescent polymer is excited by the application of a potential difference to the said film.

10. A method according to any one of the preceding claims, for the real-time monitoring of a nucleic acid amplification process, in which the target analyte is a nucleic acid undergoing amplification.

11. A method according to any one of the preceding claims, **characterized in that** a plurality of probe molecules, arranged in a predetermined matrix, are immobilized on the surface of the said coating film.

12. A microdevice for the identification and/or quantification of a target analyte in a sample, particularly a biological sample, **characterized in that** it comprises a reaction chamber, capable of receiving a solution comprising a biological sample including target analytes bound to a fluorophore, a polymer film associated with a wall of the said chamber comprising a luminescent substance capable of inducing a phenomenon of resonant energy transfer (FRET) with the said fluorophores and a plurality of probe molecules fixed to the said coating film wherein said polymer film is selected from a polymer matrix comprising fluorescent nanocrystals dispersed in said polymer matrix, photoluminescent polymer, an electroluminescent polymer or a polymer matrix incorporating fluorescent compounds.

13. A microdevice according to Claim 12, having associated thereto a radiation source capable of emitting radiation with a wavelength such that it selectively excites the said luminescent substance and means of detecting the fluorescence signal emitted in the spectral emission region of the fluorophores bound to the target analytes.

## Patentansprüche

1. Verfahren zum Bestimmen und/oder Quantifizieren eines Zielanalyten, der in einer Probe enthalten ist, im Besonderen in einer biologischen Probe, wobei das Verfahren einen Arbeitsschritt umfasst, bei dem der Zielanalyt, der an einen Fluorophor gebunden ist, mit einem Sensormolekül in Berührung gebracht wird, das auf einem Träger immobilisiert ist, um eine bestimmte Bindung zu ermöglichen, die zwischen dem Zielanalyten und dem Sensormolekül gebildet wird, **dadurch gekennzeichnet, dass** das Sensormolekül an der Oberfläche eines Trägers fixiert ist, der mit einer Dünnschicht überzogen ist, die eine lumineszierende Substanz umfasst, die das Phänomen eines Resonanzenergietransfers (FRET) mit dem Fluorophor induzieren kann, wobei die Dünnschicht, die eine lumineszierenden Substanz umfasst, aus einer Polymermatrix in der fluoreszierende Nanokristalle dispergiert sind, aus einer Polymermatrix, in der fluoreszierende Verbindungen enthalten sind, aus einer Dünnschicht, die ein photolumineszentes Polymer enthält, sowie aus einer Dünnschicht ausgewählt wird, die ein elektrolumineszentes Polymer enthält, wobei das Verfahren zusätzlich Arbeitsschritte umfasst, in denen die Substanz des Trägers, der mit der Dünnschicht beschichtet ist, aber nicht der Fluorophor, der an den Zielanalyten gebunden ist, wahlweise erregt wird, um dadurch eine Lumineszenz der Substanz hervorzurufen und das Phänomen eines Resonanzenergietransfers mit dem Fluorophor zu induzieren, und das Fluoreszenzsignal zu bestimmen, das im Bereich der spektralen Emission von nur dem Fluorophor induziert wird, der an den Zielanalyten gebunden ist.

2. Verfahren gemäß Anspruch 1, wobei die lumineszierende Substanz fluoreszierende Nanokristalle umfasst, die in einer Polymermatrix dispergiert sind, **dadurch gekennzeichnet, dass** die Polymermatrix von einem polymeren Material gebildet wird, das optisch inaktiv und im Spektralbereich vom nahen UV- bis zum Infrarot-Bereich transparent ist.

3. Verfahren gemäß Anspruch 2, **dadurch gekennzeichnet, dass** die Polymermatrix, die fluoreszierende Nanokristalle aufweist, von einem polymeren Material gebildet wird, das die Eigenschaften eines elektronischen Resists besitzt.

4. Verfahren gemäß irgendeinem der Ansprüche 2 oder 3, **dadurch gekennzeichnet, dass** die Polymermatrix Polymethylmethacrylat (PMMA) umfasst.

5. Verfahren gemäß irgendeinem der Ansprüche 2 bis 4, **dadurch gekennzeichnet, dass** die fluoreszierenden Nanokristalle kolloidale Halbleiter-Nanokristalle umfassen, vorzugsweise Kern/Hüllen-Nanokristalle.

6. Verfahren gemäß irgendeinem der Ansprüche 2 bis 5, **dadurch gekennzeichnet, dass** der Fluorophor eine spektrale Überlappung mit den Nanokristallen besitzt.

7. Verfahren gemäß irgendeinem der Ansprüche 2 bis 6, **dadurch gekennzeichnet, dass** die polymere Dünnschicht zwei oder mehr Arten von Nanokristallen umfasst, die bestimmte spektrale Eigenschaften einer fluoreszierenden Emission besitzen.

8. Verfahren gemäß Anspruch 7, **dadurch gekennzeichnet, dass** die Probe, die einer Analyse unterzogen werden soll, Zielanalyten umfasst, die mit einer Vielzahl von Fluorophoren markiert sind, die in bestimmten Spektralbereichen emittieren, wobei die Belags-Dünnschicht eine entsprechende Vielzahl von Arten von fluoreszierenden Nanokristallen umfasst, die so ausgewählt werden, dass jede Art eines Nanokristalls mit einem entsprechenden Fluorophor das Phänomen eines Resonanzenergietransfers (FRET) induzieren kann.

9. Verfahren gemäß Anspruch 1, **dadurch gekennzeichnet, dass** das elektrolumineszente Polymer dadurch erregt wird, dass an die Dünnschicht eine Potentialdifferenz angelegt wird.

10. Verfahren gemäß irgendeinem der bisherigen Ansprüche für eine Echtzeit-Beobachtung eines Nucleinsäure-Amplifikationsverfahrens, wobei der Zielanalyt eine Nucleinsäure ist, die amplifiziert wird.

11. Verfahren gemäß irgendeinem der bisherigen Ansprüche, **dadurch gekennzeichnet, dass** eine Vielzahl von Sensormolekülen, die in einer vorgegebenen Matrix angeordnet sind, auf der Oberfläche der Belags-Dünnschicht immobilisiert wird.

12. Mikrovorrichtung zum Bestimmen und/oder Quantifizieren eines Zielanalyten in einer Probe, im Besondern in einer biologischen Probe, **dadurch gekennzeichnet, dass** die Vorrichtung eine Reaktionskammer, die eine Lösung aufnehmen kann, die eine biologische Probe enthält, die Zielanalyten aufweist, die an einen Fluorophor gebunden sind, eine polymere Dünnschicht, die einer Wand der Kammer zugeordnet ist, die eine lumineszierende Substanz umfasst, die das Phänomen eines Resonanzenergietransfers (FRET) mit den Fluorophoren induzieren kann, sowie eine Vielzahl von Sensormolekülen umfasst, die an der Belags-Dünnschicht fixiert sind, wobei die polymere Dünnschicht aus einer Polymermatrix, die fluoreszierende Nanokristalle umfasst, die in der Polymermatrix dispergiert sind, aus einem photolumineszenten Polymer, aus einem elektrolumineszenten Polymer oder aus einer Polymermatrix ausgewählt wird, die fluoreszierende Verbindungen einschließt.

13. Mikrovorrichtung gemäß Anspruch 12, der eine Strahlungsquelle zugeordnet ist, die eine Strahlung mit einer Wellenlänge emittieren kann, die so beschaffen ist, dass sie die lumineszierende Substanz wahlweise erregt, sowie eine Einrichtung zugeordnet ist, um das Fluoreszenzsignal zu bestimmen, das im Bereich eines spektralen Emissionsbereiches der Fluorophore emittiert wird, die an den Zielanalyten gebunden sind.

## Revendications

1. Procédé pour l'identification et/ou la quantification d'un analyte cible présent dans un échantillon, en particulier en échantillon biologique, comprenant l'opération consistant à porter ledit analyte cible, lié à un fluorophore, en contact avec une molécule de sonde immobilisée sur un support, pour permettre la formation d'une liaison spécifique entre ledit analyte cible et ladite molécule de sonde, **caractérisé en ce que** ladite molécule de sonde est fixée à la surface d'un support revêtu d'un film comprenant une substance luminescente capable d'induire un phénomène de transfert d'énergie par résonance (FRET) avec ledit fluorophore, dans lequel ledit film comprenant une substance luminescente est choisi parmi une matrice polymère dans laquelle sont dispersés des nanocristaux fluorescents, une matrice polymère dans laquelle sont incorporés des composés fluorescents, un film comprenant un polymère photoluminescent et un film comprenant un polymère électroluminescent, et comprenant de plus les opérations consistant à exciter sélectivement ladite substance du film de revêtement de support, mais pas le fluorophore lié audit analyte cible, de façon à provoquer ainsi la luminescence de ladite substance et à induire ledit phénomène de transfert d'énergie par résonance avec ledit fluorophore, et à détecter le signal de fluorescence induit dans la région d'émission spectrale uniquement du fluorophore lié à l'analyte cible.

2. Procédé selon la revendication 1, dans lequel ladite substance luminescente comprend des nanocristaux fluorescents dispersés dans une matrice polymère, **caractérisé en ce que** ladite matrice polymère est formée d'un matériau polymère qui n'est pas optiquement actif et qui est transparent dans le champ spectral allant des UV proches aux infrarouges.

3. Procédé selon la revendication 2, **caractérisé en ce que** ladite matrice polymère contenant des nanocristaux fluorescents est formée d'un matériau polymère ayant des propriétés de réserve électronique.

4. Procédé selon l'une quelconque des revendications 2 et 3, **caractérisé en ce que** ladite matrice polymère comprend du poly(méthacrylate de méthyle) (PMMA).

5. Procédé selon l'une quelconque des revendications 2 à 4, **caractérisé en ce que** lesdits nanocristaux fluorescents comprennent des nanocristaux semiconducteurs colloïdaux, de préférence de type coeur-gaine.

6. Procédé selon l'une quelconque des revendications 2 à 5, **caractérisé en ce que** ledit fluorophore a un chevauchement spectral avec lesdits nanocristaux.

7. Procédé selon l'une quelconque des revendications 2 à 6, **caractérisé en ce que** ledit film polymère comprend deux ou plus de deux types de nanocristaux ayant des caractéristiques spectrales d'émission fluorescente différentes.

8. Procédé selon la revendication 7, **caractérisé en ce que** l'échantillon soumis à une analyse comprend des analytes cibles marqués avec une pluralité de fluorophores qui émettent dans des régions spectrales distinctes, et dans lequel ledit film de revêtement comprend une pluralité correspondante de types de nanocristaux fluorescents, choisis de telle façon que chaque type de nanocristal soit capable d'induire un phénomène de transfert d'énergie par résonance (FRET) avec un fluorophore correspondant.

9. Procédé selon la revendication 1, **caractérisé en ce que** ledit polymère électroluminescent est excité par l'application d'une différence de potentiel audit film.

10. Procédé selon l'une quelconque des revendications précédentes, pour la surveillance en temps réel d'un processus d'amplification d'acide nucléique, dans lequel l'analyte cible est un acide nucléique subissant une amplification.

11. Procédé selon l'une quelconque des revendications précédentes, **caractérisé en ce qu'**une pluralité de molécules de sonde, agencées en une matrice prédéterminée, sont immobilisées sur la surface dudit film de revêtement.

12. Microdispositif pour l'identification et/ou la quantification d'un analyte cible dans un échantillon, en particulier un échantillon biologique, **caractérisé en ce qu'**il comprend une chambre de réaction, capable de recevoir une solution comprenant un échantillon biologique contenant des analytes cibles liés à un fluorophore, un film polymère associé à une paroi de ladite chambre comprenant une substance luminescente capable d'induire un phénomène de transfert d'énergie par résonance (FRET) avec lesdits fluorophores, et une pluralité de molécules de sonde fixées audit film de revêtement, dans lequel ledit film polymère est choisi parmi une matrice polymère comprenant des nanocristaux fluorescents dispersés dans ladite matrice polymère, un polymère photoluminescent, un polymère électroluminescent, ou une matrice polymère incorporant des composés fluorescents.

13. Microdispositif selon la revendication 12, auquel sont associés une source d'irradiation capable d'émettre un rayonnement ayant une longueur d'onde telle qu'elle excite sélectivement ladite substance luminescente, et des moyens pour détecter le signal de fluorescence émis dans la région d'émission spectrale des fluorophores liés aux analytes cibles.
